# EUROPÄISCHE PATENTSCHRIFT

(11) **EP 2 921 487 B1**
(45) Veröffentlichungstag und Bekanntmachung des Hinweises auf die Patenterteilung: **03.01.2018**
(21) Anmeldenummer: 15000515.5
(22) Anmeldetag: 23.02.2015
(51) Int. Cl.: C07D 307/91

(54) **4,6-Difluor-dibenzofuran-Derivate**
4,6-Difluoro-dibenzofurans
Dibenzofuranes- 4,6-difluorés

(30) Priorität: 17.03.2014 DE 102014003600
(43) Veröffentlichungstag der Anmeldung: 23.09.2015
(73) Patentinhaber: Merck Patent GmbH, 64293 Darmstadt (DE)
(72) Erfinder: Reiffenrath, Volker, 64380 Rossdorf (DE); Bremer, Matthias, 64295 Darmstadt (DE); Fortte, Rocco, 65933 Frankfurt am Main (DE); Hirschmann, Harald, 64291 Darmstadt (DE); Engel, Martin, 64291 Darmstadt (DE)

(56) Entgegenhaltungen:
- EP-A1- 1 752 510
- WO-A1-02/055463
- WO-A1-2015/090506
- DE-A1-102005 012 585
- DATABASE REGISTRY [Online] CHEMICAL ABSTRACTS SERVICE, COLUMBUS, OHIO, US; 6. August 2002 (2002-08-06), XP002739320, gefunden im STN Database accession no. 442637-95-8

## Beschreibung

Die vorliegende Erfindung betrifft 4,6-Difluor-dibenzofuran-Derivate, ein Verfahren zu ihrer Herstellung, flüssigkristalline Medien enthaltend diese Derivate sowie elektrooptische Anzeigeelemente enthaltend diese flüssigkristallinen Medien. Die Verbindungen besitzen eine negative dielektrische Anisotropie.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete für herkömmliche Mischungen sind insbesondere Anzeigen für Uhren und Taschenrechner sowie große Anzeigetafeln, wie sie in Bahnhöfen, Flughäfen und Sportarenen verwendet werden. Weitere Anwendungsgebiete sind Displays von tragbaren und stationären Computern, Navigationssystemen und Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante ε_{∥} parallel zu den Moleküllängsachsen größer als die Dielektrizitätskonstante ε_{⊥} senkrecht zu den Moleküllängsachsen, so ist die dielektrische Anisotropie Δε = ε_{∥} - ε_{⊥} größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen anzustreben. Schwierigkeiten bereitet jedoch noch die starke Blickwinkelabhängigkeit bei Flüssigkristallanzeigen, wie sie beispielsweise für Laptops verwendet werden. Die beste Abbildungsqualität lässt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildungsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie Δε ist in diesem Fall negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

In der Druckschriften WO 02/055463, DE 102005012585 und EP 1752510 werden Dibenzofuranderivate für die Verwendung als flüssigkristallines Material offenbart. Die Verbindungen unterscheiden sich von den erfindungsgemäßen Verbindungen in der Substitution der Dibenzofuranstruktur. Die Druckschriften offenbare keine physikalischen Daten zu vergleichbaren Verbindungen.

Eine Aufgabe der vorliegenden Erfindung ist es, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz in flüssigkristallinen Medien für VA-Displays. Unabhängig von der dem Displaytyp entsprechenden dielektrischen Anisotropie sind Verbindungen gewünscht, die eine günstige Kombination der anwendungstechnischen Parameter aufweisen. Unter diesen gleichzeitig zu optimierenden Parametern sind vor allem zu nennen ein hoher Klärpunkt, eine geringe Rotationsviskosität, eine optische Anisotropie im Anwendungsintervall, sowie die Eigenschaften, die zur Erzielung von Mischungen mit den gewünschten flüssigkristallinen Phasen über einen breiten Temperaturbereich dienen (niedriger Schmelzpunkt, gute Mischbarkeit mit anderen flüssigkristallinen Komponenten der gewünschten Art).

Diese Aufgabe wird erfindungsgemäß gelöst durch Verbindungen der allgemeinen Formel I 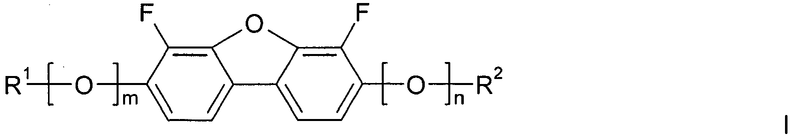 worin
- m und n: jeweils 1 sind,
- R¹ und R²: unabhängig voneinander, einen unsubstituierten Alkylrest mit 1 bis 15 Kohlenstoffatomen oder einen Alkenyl- oder Alkinylrest mit 2 bis 15 C-Atomen, welche jeweils optional ein- oder mehrfach halogeniert sind,
bedeuten,
mit der Maßgabe, dass eine Verbindung der Formel 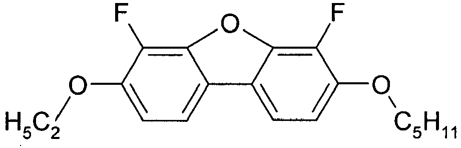 vom Stoffschutz ausgenommen ist, um die Erfindung gegenüber dem europäischen Teil der WO 2009/072722 abzugrenzen.

Die Verbindungen besitzen ein ausgeprägt negatives Δε und eignen sich daher insbesondere für eine Verwendung in VA-TFT-Displays. Vorzugsweise besitzen die erfindungsgemäßen Verbindungen ein Δε ≤ -4 und besonders bevorzugt ein Δε ≤ -8. Sie zeigen eine gute Mischbarkeit mit den üblichen, in Flüssigkristallmischungen für Displays verwendeten Substanzen, d. h. sie besitzen eine gute Löslichkeit darin. Die Rotationsviskositäten der Verbindungen und der resultierenden flüssigkristallinen Mischungen sind vorteilhaft klein.

Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die flüssigkristallinen Medien, die diese Verbindungen enthalten, weisen insbesondere eine ausreichende Breite der nematischen Phase und eine gute Tieftemperatur- und Langzeitstabilität sowie ausreichend hohe Klärpunkte auf. Die niedrigen Schmelzpunkte geben einen Hinweis auf das vorteilhafte Mischungsverhalten. Ferner weisen die erfindungsgemäßenVerbindungen der Formel I insbesondere für die Verwendung in VA-TFT-Displays geeignete Werte der optischen Anisotropie Δn auf. Bevorzugt besitzen die erfindungsgemäßen Verbindungen ein Δn von größer als 0,15 und kleiner als 0,25.

Bevorzugt bedeuten R¹ und R² jeweils unabhängig voneinander einen Alkylrest oder Alkenylrest mit 1 bis 7 bzw. 2 bis 7 Kohlenstoffatomen. Besonders bevorzugt sind R¹ und R² unabhängig voneinander in der allgemeinen Formel I ein Alkylrest mit 2 bis 5 C-Atomen. Dabei sind die Reste R¹ und R² bevorzugt verschieden.

R¹ bedeutet vorzugsweise eine Alkyl- oder Alkenylgruppe, besonders bevorzugt eine Alkylgruppe mit 1-7 C-Atomen, besonders bevorzugt mit 2 bis 5 C-Atomen. R² bedeutet vorzugsweise eine Alkyl- oder Alkenylgruppe, besonders bevorzugt eine Alkylgruppe mit 1-7 C-Atomen, besonders bevorzugt mit 2 bis 5 C-Atomen. Die Summe der Anzahl der Kohlenstoffatome in R¹ und R² zusammen beträgt bevorzugt 4, 5, 6, 7, 8, 9 oder 10, besonders bevorzugt 5, 6, 7, 8, oder 9.

Sofern R¹ und R² in Formel I jeweils unabhängig voneinander einen Alkylrest darstellen, sind diese geradkettig oder verzweigt. Vorzugsweise ist jeder dieser Reste geradkettig, hat 1, 2, 3, 4, 5, 6 oder 7 C-Atome und ist demnach vorzugsweise Methyl, Ethyl, Propyl, Butyl, Pentyl, Hexyl oder Heptyl.

R¹ und R² in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-en yl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im Allgemeinen sind die jeweiligen E-Isomeren bevorzugt. Unter den Alkenylresten sind besonders bevorzugt Prop-2-enyl, But-2- oder But-3-enyl, und Pent-3- oder Pent-4-enyl.

R¹ und R² in Formel I können unabhängig voneinander auch ein Alkinylrest mit 2 bis 15 C-Atomen sein, der geradkettig oder verzweigt ist und wenigstens eine C-C-Dreifachbindung aufweist. Bevorzugt ist 1- oder 2 Propinyl und 1-, 2- oder 3- Butinyl.

Halogen bedeutet im Zusammenhang der vorliegenden Erfindung Fluor, Chlor, Brom und Iod, insbesondere Fluor oder Chlor.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten, gesättigten, aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen.

Besonders bevorzugt sind erfindungsgemäßen Verbindungen der Formel I ausgewählt aus der Unterformel IA 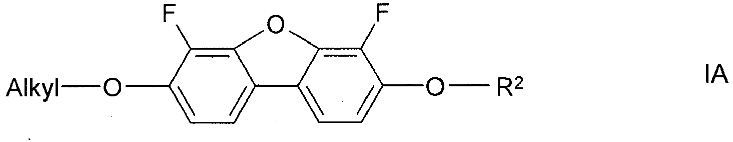 worin Alkyl einen Alkylrest mit 1 bis 15 Kohlenstoffatomen bedeutet, und R² die Bedeutungen wie für die Formel I definiert besitzt. Der Alkylrest ist bevorzugt unverzweigt (n-Alkyl). Er hat bevorzugt 1 bis 7 Kohlenstoffatome.

Besonders bevorzugte Verbindungen der Formel Isind die der Formeln: 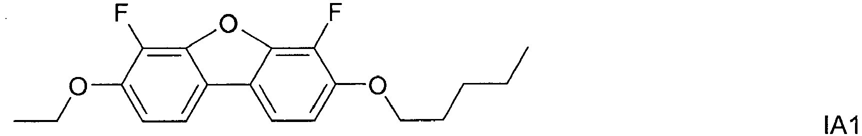 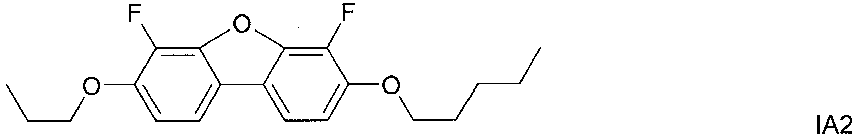 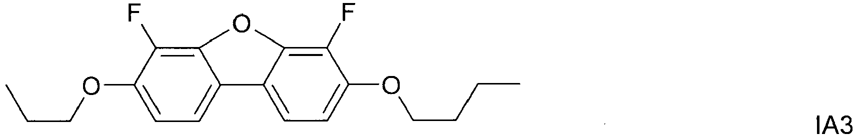 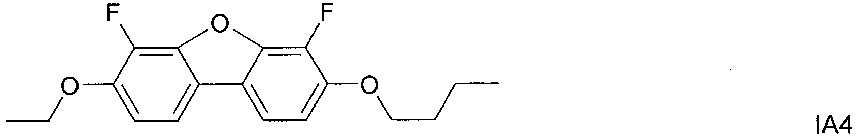 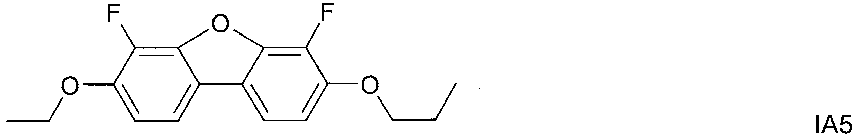 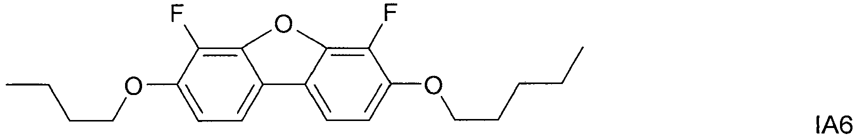 darunter besonders bevorzugt die Verbindungen der Formeln IA1 bis IA3, mit der Maßgabe, dass die Verbindung der Formel IA1 als solche vom Stoffschutz ausgenommen ist.

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Der 1,4-substituierte Cyclohexylring der Formel 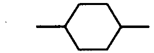 oder -Cyc-
ist in den offenbarten Verbindungen für flüssigkristalline Medien bevorzugt trans-konfiguriert, d.h. die beiden Substituenten befindend sich in der thermodynamisch bevorzugten Sesselkonformation beide in äquatorialer Position.

Die Verbindungen der allgemeinen Formel I können nach an sich bekannten Methoden dargestellt werden, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch *in situ* gebildet werden, derart, dass man sie aus dem Reaktionsgemisch nicht isoliert, sondern sofort weiter zu den Verbindungen der allgemeinen Formel I umsetzt.

Die Synthesen erfindungsgemäßer Verbindungen der allgemeinen Formel I werden in den Beispielen exemplarisch beschrieben. Die Ausgangssubstanzen sind nach allgemein zugänglichen Literaturvorschriften oder käuflich zu erhalten.

Besonders geeignete Synthesewege zu den erfindungsgemäßen Verbindungen werden im Folgenden anhand der Schemata 1, 2, 3 und 4 erläutert. Die Substituenten R¹, R² und die Zähler m und n besitzen in den folgenden Schemata die Bedeutungen wie für die Formel I.

Die Synthese der Verbindungen der Formel I mit zwei Alkoxygruppen (R1, R²) erfolgt ausgehend von der Grundverbindung Dibenzofuran (vgl. **Schema 1**).

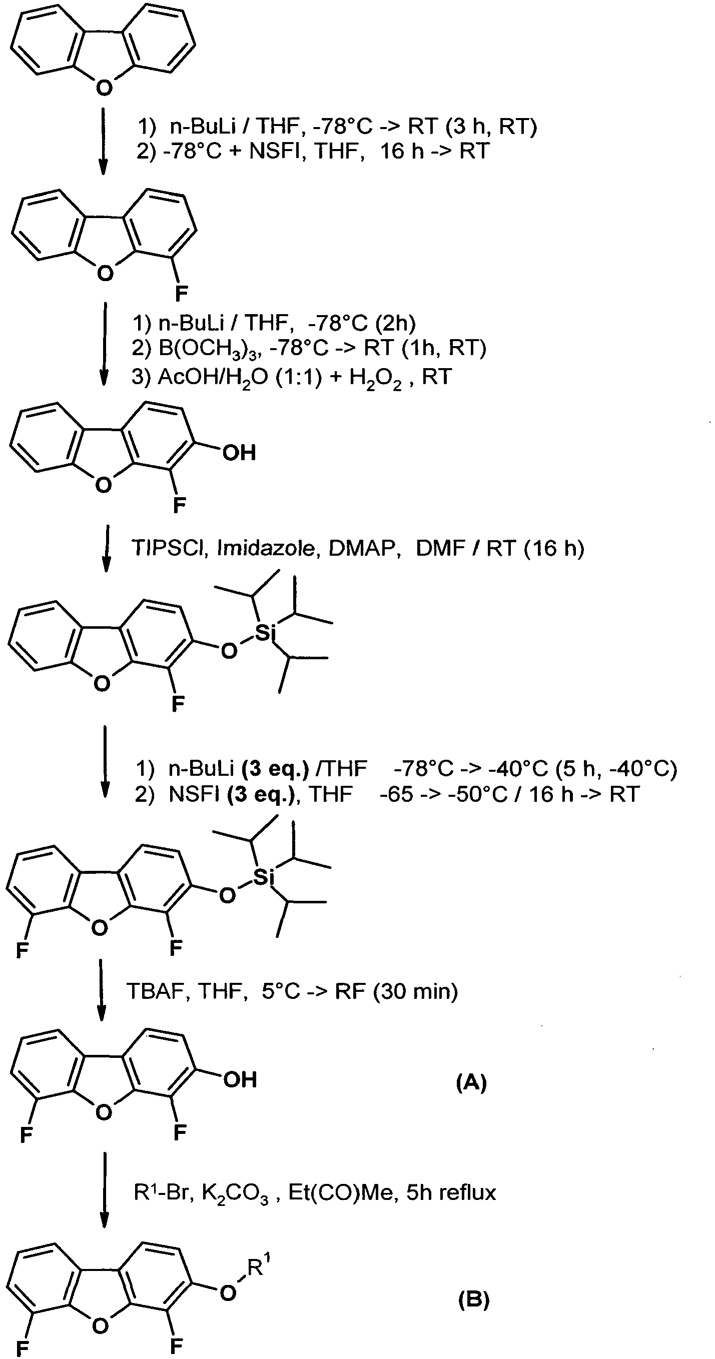

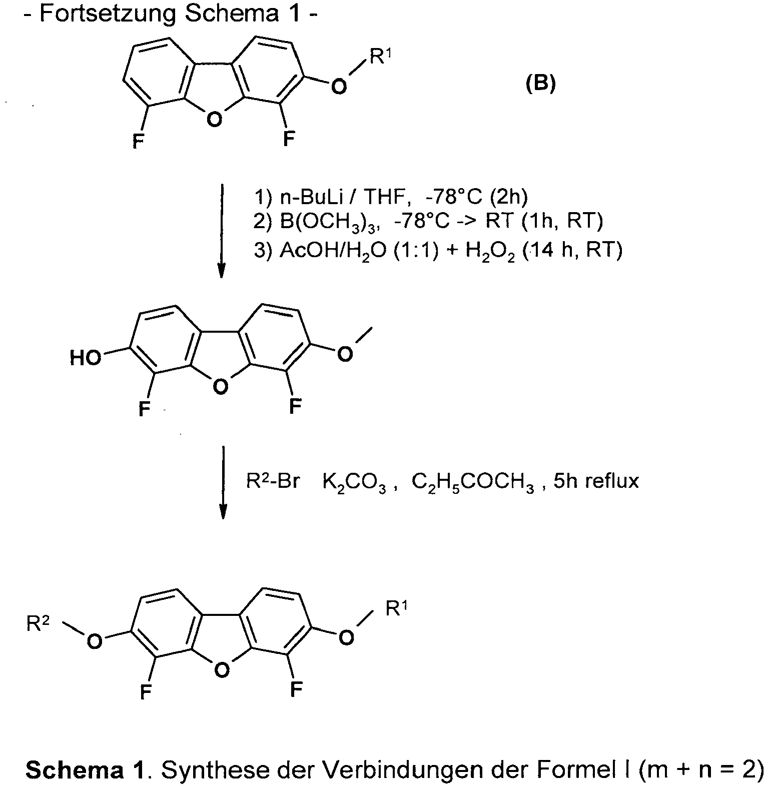

Eine alternative Synthese der Verbindungen der Formel I ergibt sich aus dem folgenden **Schema 2.**

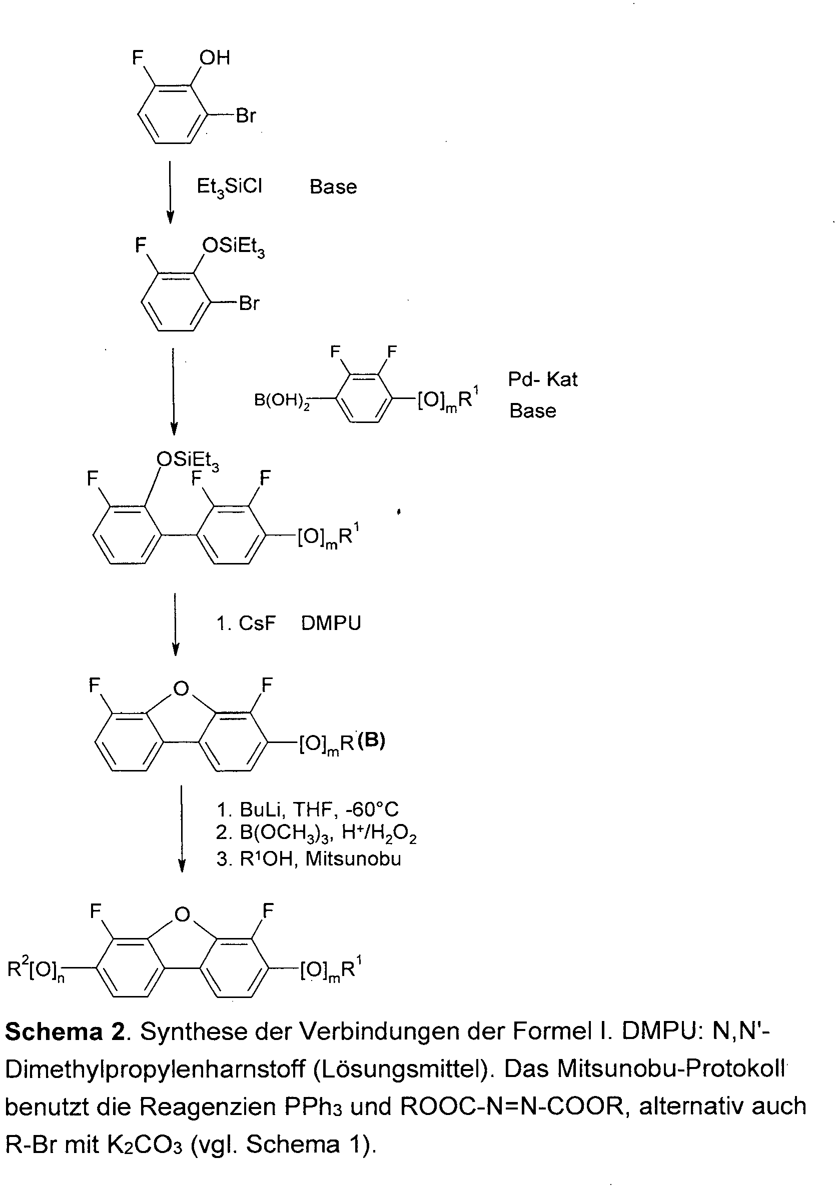

Die dargestellten Reaktionsschemata 1 bis 2 sind nur als beispielhaft aufzufassen. Der Fachmann kann entsprechende Variationen der vorgestellten Synthesen vornehmen, sowie auch andere geeignete Synthesewege beschreiten, um Verbindungen der Formel I zu erhalten.

Gemäß den zuvor dargestellten Synthesen umfasst die vorliegende Erfindung in einer Ausführungsform auch ein oder mehrere Verfahren zur Herstellung von Verbindungen der Formel I.

Die Erfindung umfasst somit ein Verfahren zur Herstellung von Verbindungen der Formel I, das dadurch gekennzeichnet ist, dass es einen Verfahrensschritt umfasst, wobei eine Verbindung der Formel (B) 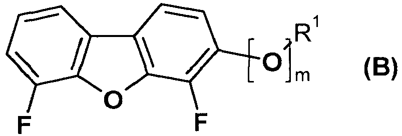 worin m, R¹ unabhängig wie in Formel I definiert sind,
an der Position 3 mittels eines Deprotonierungsreagenzes deprotoniert und zu einer Verbindung der Formel (C) 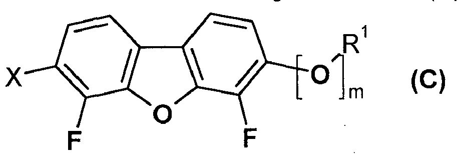 worin unabhängig
m, R¹ wie in Formel I definiert sind,
X B(OR)₂, -C(OH)R oder OH bedeutet, und
R einen Alkylrest mit 1 bis 14 C-Atomen bedeutet,
umgesetzt wird, und in einem oder mehreren weiteren Verfahrensschritten zu einer Verbindung der Formel I umgesetzt wird.

Die verschiedenen Gruppen X in Formel (C) werden erhalten, indem man den in *ortho*-Position zum Fluoratom metallierten Aromaten mit Borsäuretrialkylester B(OR)₃ zu X= -B(OR)₂, mit Aldehyd RCHO zu -C(OH)R, und ggf. die entstandene Boronsäuregruppe X= -B(OR)₂ oxidativ (z.B. mit H₂O₂) zu OH umsetzt. Bevorzugte Bedingungen für die Metallierung sind die Umsetzung mit einem Lithiumalkyl wie n-BuLi , in THF, bei ca. -70°C, dann Zugabe des Elektrophils.

Das Verfahren und die anschließende Aufarbeitung des Reaktionsgemisches kann grundsätzlich als Batch-Reaktion oder in kontinuierlicher Reaktionsweise durchgeführt werden. Die kontinuierliche Reaktionsweise umfasst z. B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Cokristallisation oder durch Nanofiltration an Membranen.

Wie bereits erwähnt, können die Verbindungen der allgemeinen Formel I in flüssigkristallinen Medien verwendet werden. Gegenstand der vorliegenden Erfindung ist daher auch ein flüssigkristallines Medium mit mindestens zwei flüssigkristallinen Verbindungen, enthaltend mindestens eine Verbindung der allgemeinen Formel I, mit der Maßgabe, dass eine Mischung der Zusammensetzung

| Verbindung | Gew.-% |
|---|---|
| CY-3-O2 | 12,5 |
| CCY-3-O1 | 9 |
| CCY-3-O2 | 11 |
| CCY-4-O2 | 7 |
| CPY-3-O2 | 3 |
| CC-3-V | 31 |
| B-2O-O5 | 4 |
| PY-V2-O2 | 5,5 |
| CPY-V-O2 | 6 |
| CPY-V-O4 | 5 |
| CCY-V-O2 | 6 |

ausgenommen ist, um die Erfindung gegenüber dem europäischen Teil der WO 2009/072722 abzugrenzen.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydropyrane, Phenyl- oder Cyclohexylbenzoate, Cyclohexancarbonsäurephenyl- oder-cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (lila), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

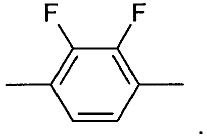

In den Verbindungen der Gruppe B, die mit den Teilformeln (IIb), (IIIb), (IVb), (Vb) und (VIb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im Folgenden als Gruppe C bezeichnet und die Verbindungen dieser Untergruppe werden entsprechend mit Teilformeln (IIc), (IIIc), (IVc), (Vc) und (VIc) beschrieben. In den Verbindungen der Teilformeln (IIc), (IIIc), (IVc), (Vc) und (VIc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:
Gruppe A:
   0 bis 90 %, vorzugsweise 20 bis 90 %, insbesondere 30 bis 90 %.
Gruppe B:
   0 bis 80 %, vorzugsweise 10 bis 80 %, insbesondere 10 bis 70 %.
Gruppe C:
   0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Die Medien enthalten vorzugsweise eine, zwei, drei, vier oder fünf erfindungsgemäße Verbindungen der Formel I.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen Δε insbesondere für eine Verwendung in VA-TFT-Displays.

Gegenstand der vorliegenden Erfindung sind daher auch elektrooptische Flüssigkristallanzeigeelemente, enthaltend ein erfindungsgemäßes flüssigkristallines Medium.

Weitere Kombinationen der Ausführungsformen und Varianten der Erfindung gemäß der Beschreibung ergeben sich aus den Ansprüchen.

Die Erfindung wird im Folgenden anhand von Ausführungsbeispielen näher erläutert, ohne dadurch jedoch eingeschränkt werden zu sollen. Der Fachmann wird in der Lage sein, den Beispielen Details zur Durchführung zu entnehmen, die in der allgemeinen Beschreibung nicht im Einzelnen aufgeführt sind, sie nach allgemeinen Fachkenntnissen zu verallgemeinern und auf seine spezielle Problemstellung anzuwenden.

Neben den üblichen und wohlbekannten Abkürzungen werden folgende Abkürzungen verwendet:
K: Kristalline Phase; N: Nematische Phase; Sm: Smektische Phase;
I: Isotrope Phase. Die Zahlen zwischen diesen Symbolen geben die Übergangstemperaturen der betreffenden Substanz wieder.

Temperaturangaben sind, soweit nichts anderes angegeben, in °C.

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Vor- und nachstehend bedeutet Δn die optische Anisotropie (589 nm, 20 °C) und Δε die dielektrische Anisotropie (1 kHz, 20 °C). Die dielektrische Anisotropie Δε wird bei 20°C und 1 kHz bestimmt. Die optische Anisotropie Δn wird bei 20°C und einer Wellenlänge von 589,3 nm bestimmt.

Die Δε- und An-Werte, der sowie die Rotationsviskosität (γ₁) der erfindungsgemäßen Verbindungen werden durch lineare Extrapolation aus flüssigkristallinen Mischungen erhalten, die zu 5 bis 10 % aus der jeweiligen erfindungsgemäßen Verbindung und zu 90-95 % aus der kommerziell erhältlichen Flüssigkristallmischung ZLI-2857 (für Δε) bzw. ZLI-4792 (für Δn, γ₁) bestehen (Mischungen Fa. Merck KGaA, Darmstadt).

Vor- und nachstehend bedeuten die Abkürzungen:
- MTBE: Methyl-tert-butylether
- THF: Tetrahydrofuran
- DMF: Dimethylformamid
- DMAP: 4-(Dimethylamino)pyridin
- NSFI: N-Fluorbenzolsulfonimid
- ges. Lsg.: gesättigte Lösung
- *n*-BuLi: *n*-Butyllithium, Lösung in Hexan
- RT: Raumtemperatur, ca. 20 °C
- TIPSCl: Triisopropylsilyl-Chlorid

### Beispiele

Die Ausgangssubstanzen können nach allgemein zugänglichen Literaturvorschriften oder käuflich erhalten werden.

### Beispiel 1: 3-Butoxy-4,6-difluor-7-propoxy-dibenzofuran

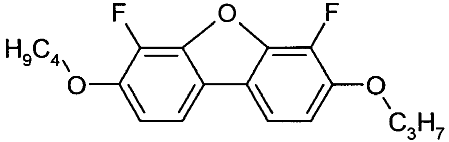

### Schritt 1

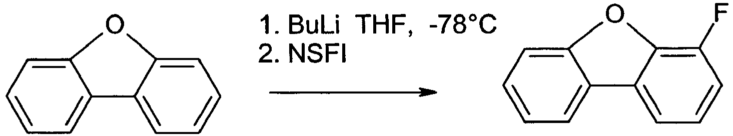

50 g Dibenzofuran werden in 1500 ml THF vorgelegt, und bei -60 bis -75°C mit 152 g 15%iger BuLi-Lsg. in Hexan tropfenweise versetzt. Man erwärmt auf RT und rührt 3 h nach. Danach kühlt man wieder auf -75 °C ab und versetzt bei -75 bis -60°C mit einer Lösung von 112,5 g N-Fluorbenzolsulfonimid in 1000 ml THF. Nach weiteren 30 min bei -70°C lässt man das Reaktionsgemisch auf Umgebungstemperatur erwärmen, hydrolysiert den Ansatz mit Wasser und arbeitet extraktiv auf.

Das Rohprodukt (rotbraunes Öl) wird chromatographisch gereinigt (Laufmittel: n-Heptan).

Weiße Kristalle.

### Schritt 2

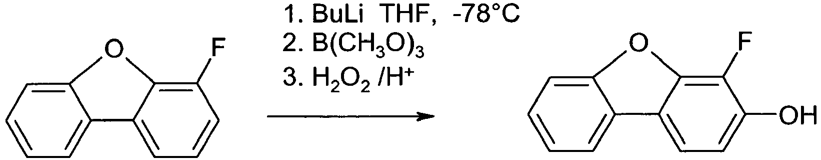

40 g 4-Fluordibenzofuran werden in 450 ml THF vorgelegt, und bei -60 bis -75°C mit 96 g 15%igeer BuLi-Lsg. in Hexan tropfenweise versetzt. Man rührt 2 Stunden nach. Danach versetzt man bei -75 bis -60°C mit einer Lösung von 25 g Trimethylborat in 80 ml THF. Nach weiteren 30 min bei -70°C lässt man das Reaktionsgemisch auf Umgebungstemperatur erwärmen, hydrolysiert den Ansatz langsam mit einem Gemisch von 30 g Eisessig und 40 ml Wasser. Anschließend tropft man 40 g 30%iges Wasserstoffperoxid so zu, dass die Temperatur 45 °C nicht übersteigt. Man rührt noch 12 h bei RT und arbeitet extraktiv auf.

Das Rohprodukt wird chromatographisch gereinigt (Lauf-mittel: n-Heptan / MTB 4/1).

Weiße Kristalle.

### Schritt 3

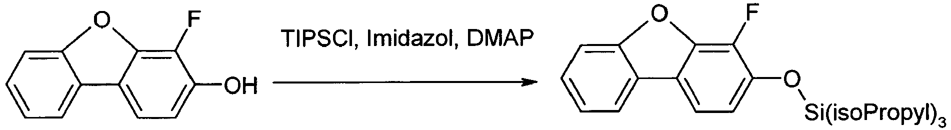

36,8 g 4-Fluor-dibenzofuran-3-ol, 13,6 g Imidazol und 24,5 g DMAP werden in 700 ml DMF gelöst und bei 25 °C mit einer Lösung von 55 g Chlortriisopropylsilan in 35 ml DMF versetzt. Man rührt noch 12 h bei RT, gießt den Ansatz vorsichtig auf Eiswasser und arbeitet extraktiv auf.

Das Rohprodukt wird chromatographisch gereinigt (Lauf-mittel: n-Heptan / Chlorbutan 9/1).

Farbloses Öl.

### Schritt 4

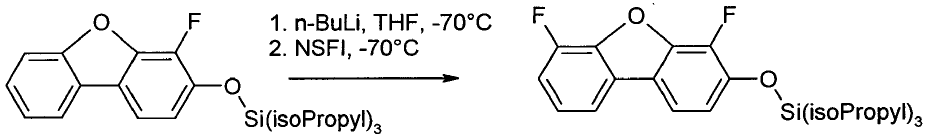

30,8 g (4-Fluor-dibenzofuran-3-yloxy)triisopropylsilan werden in 850 ml THF vorgelegt, und bei -60 bis -75°C mit 106 g (3 äqu.) 15%iger BuLiLsg. in Hexan tropfenweise versetzt. Man erwärmt auf -40 °C und rührt 5 h bei dieser Temperatur nach. Danach kühlt man wieder auf -65 °C ab und versetzt bei -65 bis -50°C mit einer Lösung von 78,8 g N-Fluorbenzolsulfonimid in 300 ml THF. Nach weiteren 30 min bei -50 °C lässt man das Reaktionsgemisch auf Umgebungstemperatur erwärmen, hydrolysiert den Ansatz mit Wasser und arbeitet extraktiv auf.

Das Rohprodukt (rotbraunes Öl) wird chromatographisch gereinigt (Laufmittel: n-Heptan / Chlorbutan 4:1) und aus Ethanol umkristallisiert.

Weiße Kristalle.

### Schritt 5

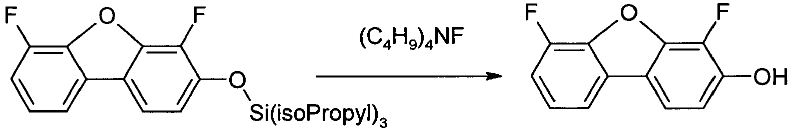

15,4 g (4,6-Difluor-dibenzofuran-3-yloxy)triisopropylsilan werden in 150 ml THF gelöst und bei 5°C mit 57 ml einer 1 M Lösung
vonTetrabutylammoniumfluorid in THF versetzt. Man rührt noch 30 min bei RT und arbeitet extraktiv auf.

Das Rohprodukt wird chromatographisch gereinigt (Laufmittel: n-Heptan / MTB 2/1). Umkristallisiert aus Heptan / Toluol 1/1,5.

Weiße Kristalle.

### Schritt 6

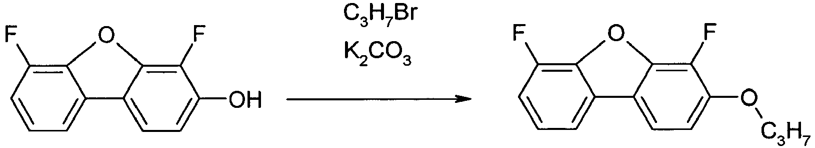

17,3 g 4,6-Difluor-dibenzofuran-3-ol werden mit 14,5 g n-Propylbromid und 16,3 g Kaliumcarbonat in 150 ml Methylethylketon und 15 h am Rückfluss gekocht. Man arbeitet extraktiv auf.

Das Rohprodukt wird chromatographisch gereinigt (Lauf-mittel: n-Heptan / MTB 4/1). Umkristallisiert aus Heptan.

Weiße Kristalle.

### Schritt 7

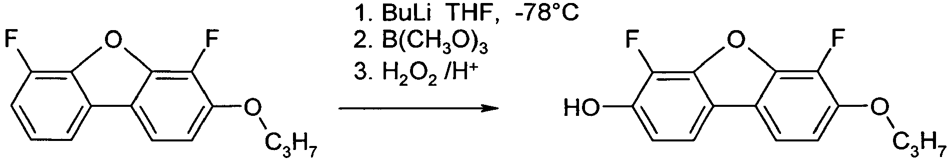

17,3 g 4,6-Difluor-3-propyloxy-dibenzofuran werden in 250 ml THF vorgelegt, und bei -60 bis -75°C mit 33 g 15%iger BuLi-Lsg. in Hexan tropfenweise versetzt. Man rührt 2 h nach. Danach versetzt man bei -75 bis -60°C mit einer Lösung von 7,9 g Trimethylborat in 20 ml THF. Nach weiteren 30 min bei -70°C lässt man das Reaktionsgemisch auf RT erwärmen, hydrolysiert den Ansatz langsam mit einem Gemisch von 10 g Eisessig und 12 ml Wasser. Anschließend tropft man 16 g 30%iges Wasserstoffperoxid so zu, dass die Temperatur 45°C nicht übersteigt. Man rührt noch 12 h bei RT und arbeitet extraktiv auf.

Das Rohprodukt wird chromatographisch gereinigt (Laufmittel: n-Heptan / MTB 3/1).

Ausbeute 9,9 g. Weiße Kristalle.

### Schritt 8

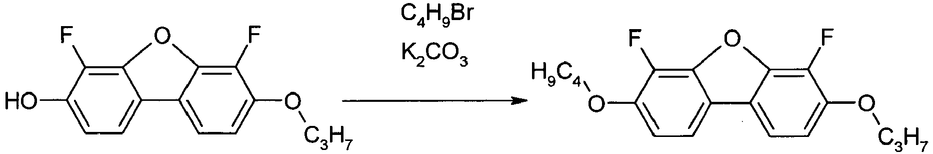

7,1 g 4,6-Difluor-7-propoxy-dibenzofuran-3-ol werden mit 7 g n-Butylbromid und 5,3 g Kaliumcarbonat in 65 ml Methylethylketon und 5 h am Rückfluss gekocht. Man arbeitet extraktiv auf.
Das Rohprodukt wird chromatographisch gereinigt (Laufmittel: n-Heptan / Chlorbutan 2/1). Umkristallisiert aus Heptan.
Ausbeute 7,9 g. Weiße Kristalle.
Phasen: K 68 I (Schmp. 68 °C, vgl. auch Tabelle).

Analog zu Beispiel 1 werden die folgenden Verbindungen hergestellt: 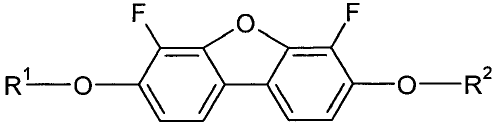 Die Reste R^{1/2} sind geradkettig, d.h. unverzweigt, soweit nicht anders angegeben. Die Stoffdaten ergeben sich aus Tabelle 1.

**Tabelle 1.**

| **R¹** | **R²** | **Schmp. [°C]** | **Δε** | **Δn** | **γ₁ [mPa·s]** | **Clp. [°C]** |
|---|---|---|---|---|---|---|
| -CH₃ | -CH₃ | | | | | |
| -CH₃ | -C₂H₅ | 119 | | | | |
| -CH₃ | -C₃H₇ | 93 | -15 | 0,193 | 149 | 59 |
| -CH₃ | -C₄H₉ | 79 | -13 | 0,191 | 144 | 60 |
| -CH₃ | -C₅H₁₁ | 79 | -14 | 0,185 | 144 | 46 |
| -CH₃ | -C₆H₁₃ | | | | | |
| -C₂H₅ | -C₂H₅ | | | | | |
| -C₂H₅ | -C₃H₇ | 85 | -15 | 0,189 | 128 | 66 |
| -C₂H₅ | -C₄H₉ | 77 | -14 | 0,189 | 116 | 62 |
| | -CH₂CH(CH₃)₂ | 90 | -14 | 0,183 | 152 | 55 |
| -C₂H₅ | -(CH₂)₂CH=CH₂ | 87 | -14 | 0,196 | 102 | 51 |
| -C₂H₅ | -(CH₂)₂CH(CH₃)₂ | 90 | -14 | 0,179 | 108 | 41 |
| -C₂H₅ | -C₅H₁₁ | 57 | -14 | 0,181 | 119 | 59 |
| -C₂H₅ | -C₆H₁₃ | 68 | -13 | 0,180 | 131 | 61 |
| -C₂H₅ | -(CH₂)₃CH(CH₃)₂ | 56 | -13 | 0,167 | 144 | 51 |
| -C₃H₇ | -C₃H₇ | 75 | -14 | 0,193 | 123 | 68 |
| -C₃H₇ | -C₄H₉ | 68 | -13 | 0,192 | 115 | 63 |
| -C₃H₇ | -C₅H₁₁ | 63 | -13 | 0,176 | 104 | 58 |
| -C₃H₇ | -C₆H₁₃ | 69 | -13 | 0,171 | 125 | 61 |
| -C₄H₉ | -C₄H₉ | 87 | -12 | 0,184 | 73 | 62 |
| -C₄H₉ | -C₅H₁₁ | 76 | -12 | 0,181 | 111 | 59 |
| -C₄H₉ | -(CH₂)₂CH=CHCH₃^{*)} | 65 | -13 | 0,187 | 116 | 36 |
| -C₄H₉ | -C₆H₁₃ | | | | | |
| -C₅H₁₁ | -CF₃ | 64 | -4 | 0,132 | 73 | -24 |
| -C₅H₁₁ | -C₅H₁₁ | | | | | |
| -C₅H₁₁ | -C₆H₁₃ | | | | | |

| | | | | | | |
|---|---|---|---|---|---|---|
| ^{*)} trans-Isomer | | | | | | |

## Patentansprüche

1. Verbindungen der Formel I: 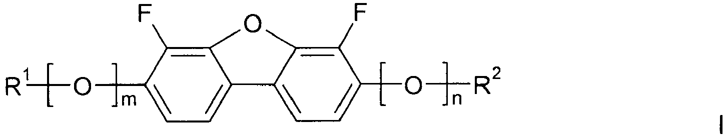 worin
m und n jeweils 1 sind, und
R¹ und R² unabhängig voneinander, einen Alkylrest mit 1 bis 15 C-Atomen oder einen Alkenyl- oder Alkinylrest mit 2 bis 15 C-Atomen bedeuten, welche jeweils optional ein- oder mehrfach halogeniert sind
mit der Maßgabe, dass eine Verbindung der Formel 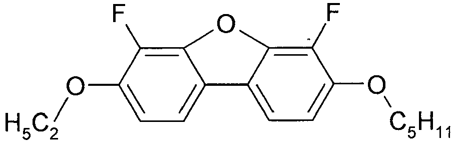 ausgenommen ist.

2. Verbindungen nach Anspruch 1, **dadurch gekennzeichnet, dass** die Summe der Anzahl der Kohlenstoffatome in R¹ und R² zusammen 4, 5, 6, 7, 8, 9 oder 10 beträgt.

3. Verbindungen nach Anspruch 1 oder 2, **dadurch gekennzeichnet, dass**
R¹ und R² unabhängig voneinander einen Alkylrest mit 1 bis 7 oder einen Alkenylrest mit 2 bis 7 Kohlenstoffatomen bedeuten.

4. Verbindungen nach einem oder mehreren der Ansprüche 1 bis 3, **dadurch gekennzeichnet dass** in Formel I.
R¹ und R² unabhängig voneinander einen Alkylrest mit 2 bis 5 C-Atomen bedeuten.

5. Verbindungen nach einem oder mehreren der Ansprüche 1 bis 4, **dadurch gekennzeichnet, dass** die Verbindungen der Formel I ausgewählt sind aus der Unterformel IA 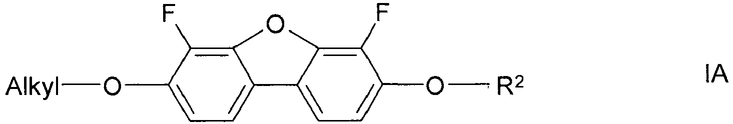 worin
Alkyl einen Alkylrest mit 1 bis 15 Kohlenstoffatomen bedeutet, und
R² die Bedeutungen wie für die Formel I definiert besitzt.

6. Verbindungen nach einem oder mehreren der Ansprüche 1 bis 5, **dadurch gekennzeichnet, dass** die Verbindungen ausgewählt sind aus Verbindungen der Formeln

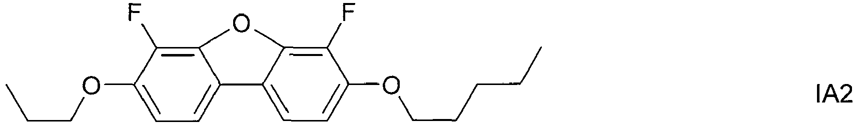

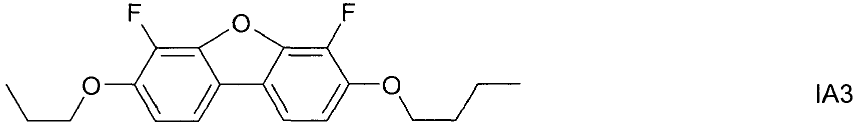

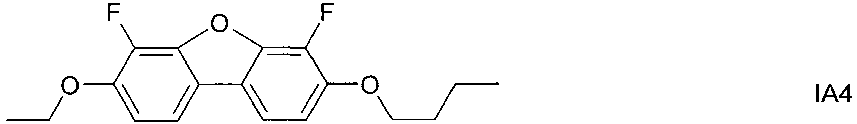

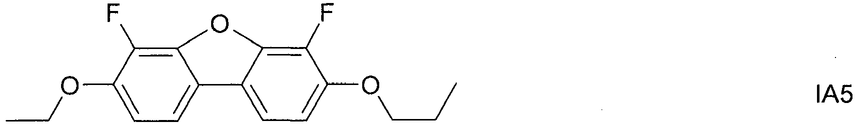

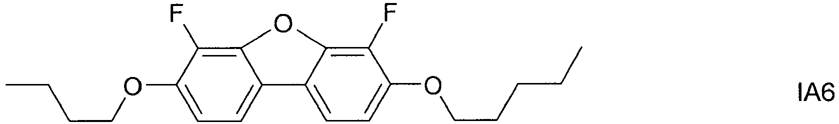

7. Verbindungen nach einem oder mehreren der Ansprüche 1 bis 6, **dadurch gekennzeichnet, dass**
R¹ und R² unabhängig voneinander einen Alkylrest mit 2 bis 6 oder einen Alkenylrest mit 3 bis 6 Kohlenstoffatomen oder CF₃ bedeuten.

8. Verwendung einer oder mehrerer Verbindungen nach einem oder mehreren der Ansprüche 1 bis 7 in flüssigkristallinen Medien.

9. Flüssigkristallines Medium enthaltend mindestens zwei Verbindungen, **dadurch gekennzeichnet, dass** es mindestens eine Verbindung der Formel I 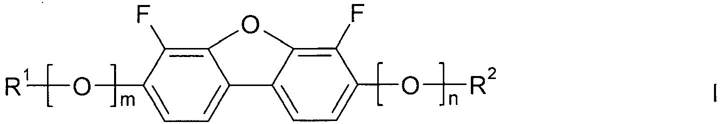 worin
m und n jeweils 1 sind, und
R¹ und R² unabhängig voneinander, einen Alkylrest mit 1 bis 15 C-Atomen oder einen Alkenyl- oder Alkinylrest mit 2 bis 15 C-Atomen bedeuten, welche jeweils optional ein- oder mehrfach halogeniert sind,
enthält
mit der Maßgabe, dass eine Mischung der Zusammensetzung
| Verbindung | Gew.-% |
|---|---|
| CY-3-O2 | 12,5 |
| CCY-3-O1 | 9 |
| CCY-3-O2 | 11 |
| CCY-4-O2 | 7 |
| CPY-3-O2 | 3 |
| CC-3-V | 31 |
| B-2O-O5 | 4 |
| PY-V2-O2 | 5,5 |
| CPY-V-O2 | 6 |
| CPY-V-O4 | 5 |
| CCY-V-O2 | 6 |
ausgenommen ist.

10. Flüssigkristallines Medium nach Anspruch 9, **dadurch gekennzeichnet, dass** in Formel I
R¹ und R² unabhängig voneinander einen Alkylrest mit 2 bis 5 C-Atomen bedeuten.

11. Flüssigkristallines Medium nach Anspruch 9, **dadurch gekennzeichnet, dass** es mindestens eine Verbindung der Formel I ausgewählt aus Verbindungen der Formeln 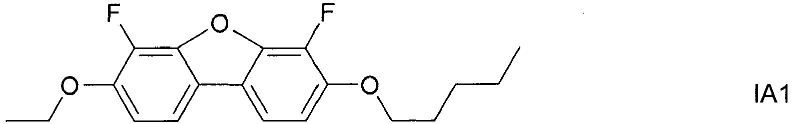 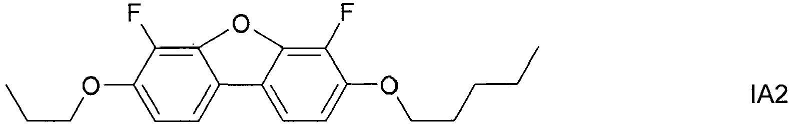 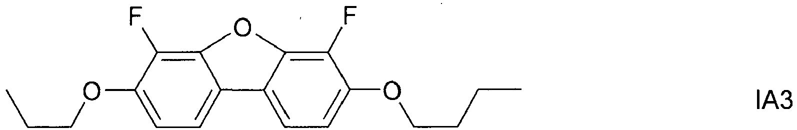 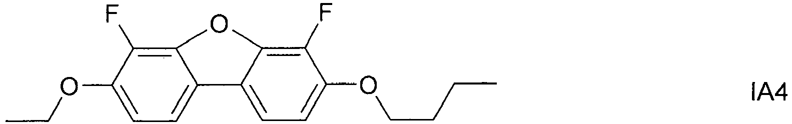 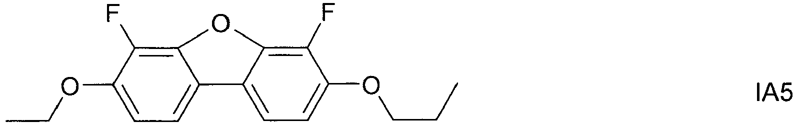 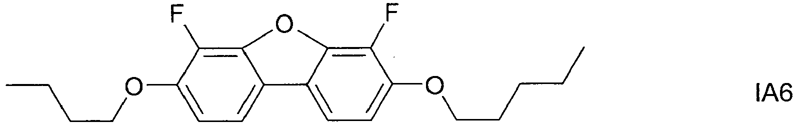 enthält.

12. Elektrooptisches Anzeigeelement, enthaltend ein flüssigkristallines Medium nach Anspruch 9.

13. Verfahren zur Herstellung von Verbindungen der Formel I nach einem oder mehreren der Ansprüche 1 bis 7, **dadurch gekennzeichnet, dass** es einen Verfahrensschritt umfasst, wobei eine Verbindung der Formel (B) 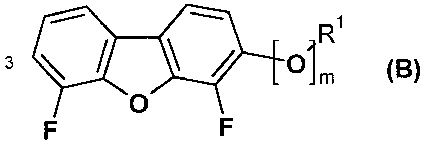 worin m, R¹ unabhängig wie in Formel I nach einem der Ansprüche 1 bis 7 definiert sind,
an der Position 3 mittels eines Deprotonierungsreagenzes deprotoniert und zu einer Verbindung der Formel (C) 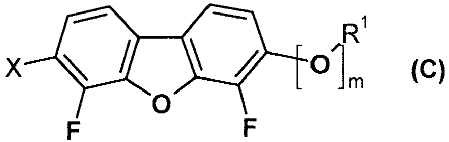 worin unabhängig
m, R¹ wie in Formel I definiert sind,
X B(OR)₂, -C(OH)R oder OH bedeutet, und
R einen Alkylrest mit 1 bis 14 C-Atomen bedeutet,
umgesetzt wird, und in einem oder mehreren weiteren Verfahrensschritten zu einer Verbindung der Formel I umgesetzt wird.

## Claims

1. Compounds of the formula I: 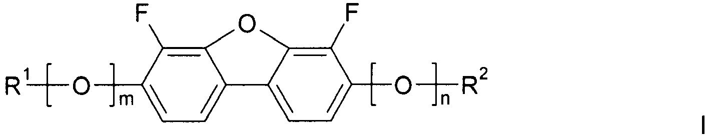 in which
m and n are each 1, and
R¹ and R², independently of one another, denote an alkyl radical having 1 to 15 C atoms or an alkenyl or alkynyl radical having 2 to 15 C atoms, which are in each case optionally mono- or polyhalogenated, 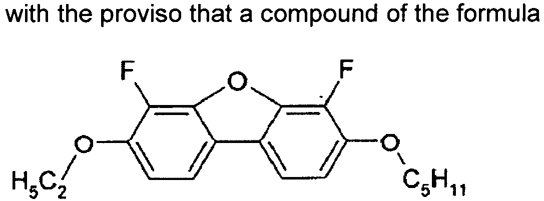
is excluded.

2. Compounds according to Claim 1, **characterised in that** the sum of the number of carbon atoms in R¹ and R² together is 4, 5, 6, 7, 8, 9 or 10.

3. Compounds according to Claim 1 or 2, **characterised in that**
R¹ and R², independently of one another, denote an alkyl radical having 1 to 7 carbon atoms or an alkenyl radical having 2 to 7 carbon atoms.

4. Compounds according to one or more of Claims 1 to 3, **characterised in that**, in formula I,
R¹ and R², independently of one another, denote an alkyl radical having 2 to 5 C atoms.

5. Compounds according to one or more of Claims 1 to 4, **characterised in that** the compounds of the formula I are selected from the sub-formula IA 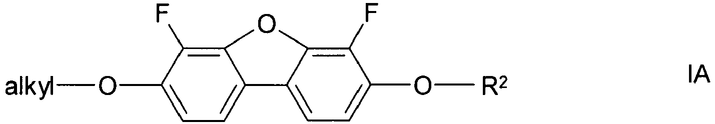 in which
alkyl denotes an alkyl radical having 1 to 15 carbon atoms, and
R² has the meanings as defined for the formula I.

6. Compounds according to one or more of Claims 1 to 5, **characterised in that** the compounds are selected from compounds of the formulae

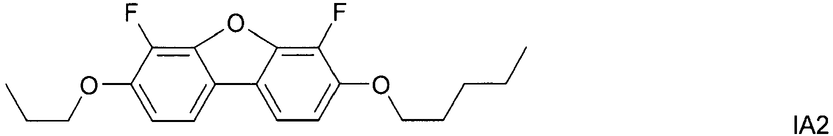

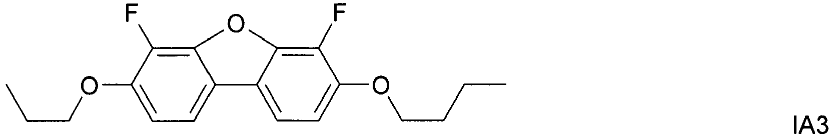

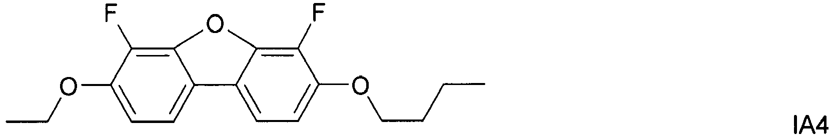

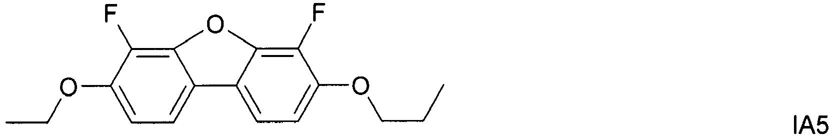

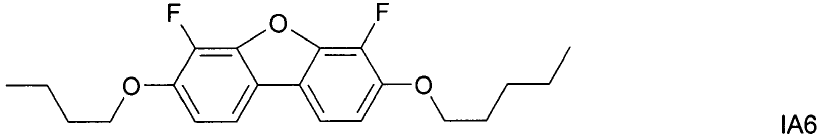

7. Compounds according to one or more of Claims 1 to 6, **characterised in that**
R¹ and R², independently of one another, denote an alkyl radical having 2 to 6 carbon atoms or an alkenyl radical having 3 to 6 carbon atoms or CF₃.

8. Use of one or more compounds according to one or more of Claims 1 to 7 in liquid-crystalline media.

9. Liquid-crystalline medium comprising at least two compounds, **characterised in that** it comprises at least one compound of the formula I 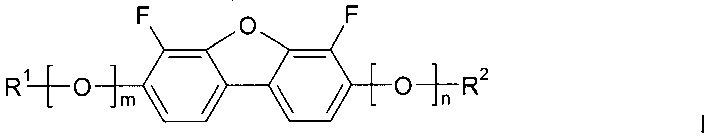 in which
m and n are each 1, and
R¹ and R², independently of one another, denote an alkyl radical having 1 to 15 C atoms or an alkenyl or alkynyl radical having 2 to 15 C atoms, which are in each case optionally mono- or polyhalogenated,
with the proviso that a mixture of the composition
| Compound | % by weight |
|---|---|
| CY-3-O2 | 12.5 |
| CCY-3-O1 | 9 |
| CCY-3-O2 | 11 |
| CCY-4-O2 | 7 |
| CPY-3-O2 | 3 |
| CC-3-V | 31 |
| B-2O-O5 | 4 |
| PY-V2-O2 | 5.5 |
| CPY-V-O2 | 6 |
| CPY-V-O4 | 5 |
| CCY-V-O2 | 6 |
is excluded.

10. Liquid-crystalline medium according to Claim 9, **characterised in that**, in formula I,
R¹ and R², independently of one another, denote an alkyl radical having 2 to 5 C atoms.

11. Liquid-crystalline medium according to Claim 9, **characterised in that** it comprises at least one compound of the formula I selected from compounds of the formulae

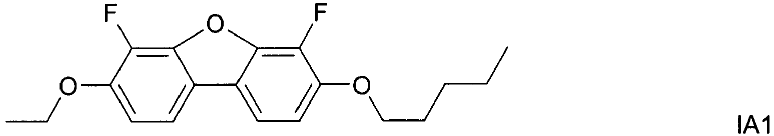

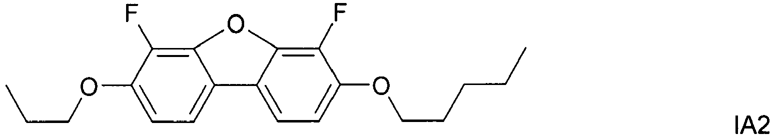

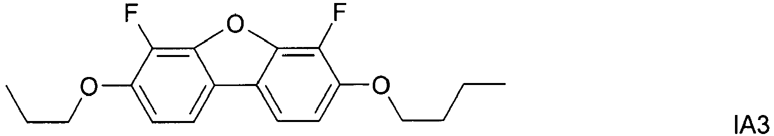

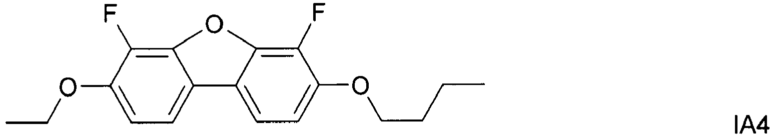

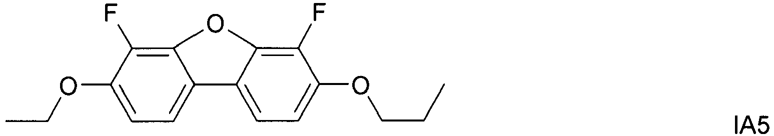

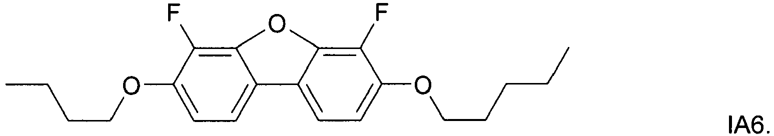

12. Electro-optical display element containing a liquid-crystalline medium according to Claim 9.

13. Process for the preparation of compounds of the formula I according to one or more of Claims 1 to 7, **characterised in that** it includes a process step in which a compound of the formula (B) 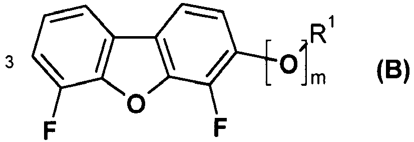 in which m, R¹ independently are defined as in formula I according to one of Claims 1 to 7,
is deprotonated at position 3 by means of a deprotonating reagent and converted into a compound of the formula (C) 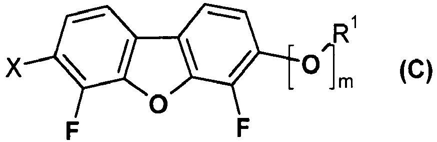 in which, independently,
m, R¹ are defined as in formula I,
X denotes B(OR)₂, -C(OH)R or OH, and
R denotes an alkyl radical having 1 to 14 C atoms,
and in one or more further process steps is converted into a compound of the formula I.

## Revendications

1. Composés de la formule I : 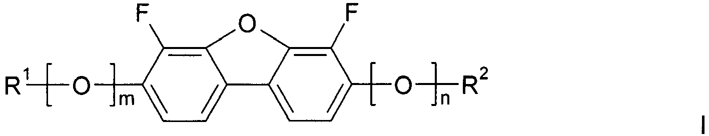 formule dans laquelle :
m et n sont chacun 1 ; et
R¹ et R² représentent, indépendamment l'un de l'autre, un radical alkyle qui comporte 1 à 15 atome(s) de C ou un radical alkényle ou alkynyle qui comporte 2 à 15 atomes de C, lesquels sont dans chaque cas en option monohalogénés ou polyhalogénés ;
étant entendu qu'un composé de la formule 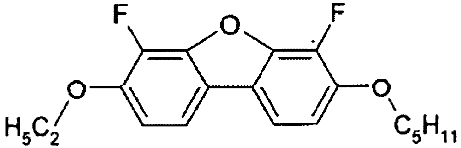 est exclus.

2. Composés selon la revendication 1, **caractérisés en ce que** la somme du nombre d'atomes de carbone dans R¹ et dans R² en association est égale à 4, 5, 6, 7, 8, 9 ou 10.

3. Composés selon la revendication 1 ou 2, **caractérisés en ce que**
R¹ et R² représentent, indépendamment l'un de l'autre, un radical alkyle qui comporte 1 à 7 atome(s) de carbone ou un radical alkényle qui comporte 2 à 7 atomes de carbone.

4. Composés selon une ou plusieurs des revendications 1 à 3, **caractérisés en ce que**, dans la formula I :
R¹ et R² représentent, indépendamment l'un de l'autre, un radical alkyle qui comporte 2 à 5 atomes de C.

5. Composés selon une ou plusieurs des revendications 1 à 4, **caractérisés en ce que** les composés de la formule I sont sélectionnés parmi la sous-formule IA : 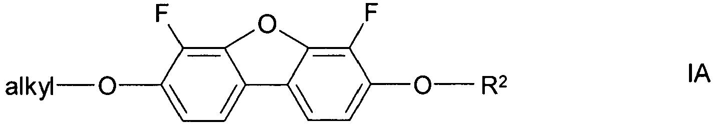 sous-formule dans laquelle :
alkyl représente un radical alkyle qui comporte 1 à 15 atome(s) de carbone ; et
R² présente les significations telles que définies pour la formule I.

6. Composés selon une ou plusieurs des revendications 1 à 5, **caractérisés en ce que** les composés sont sélectionnés parmi les composés des formules :

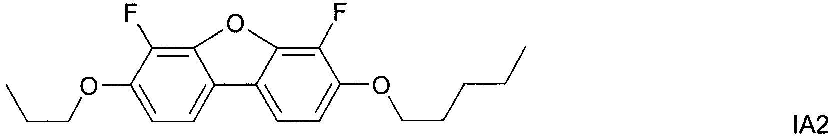

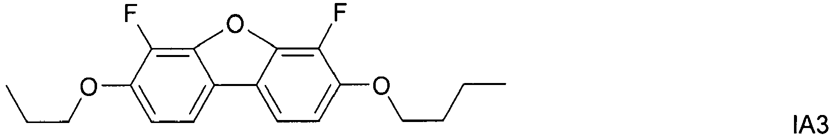

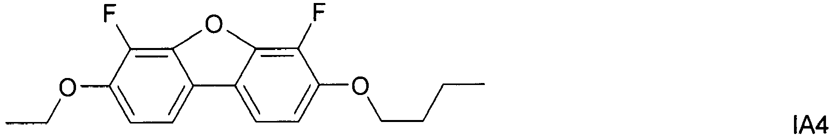

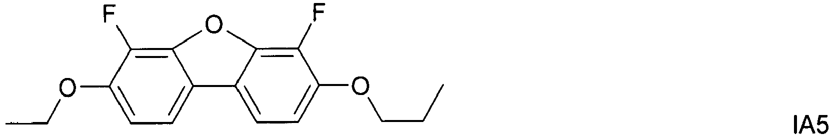

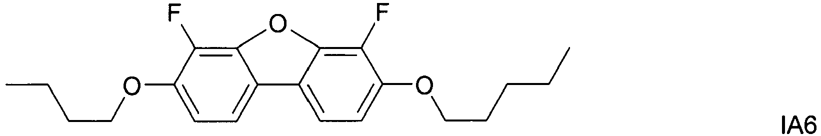

7. Composés selon une ou plusieurs des revendications 1 à 6, **caractérisés en ce que** :
R¹ et R² représentent, indépendamment l'un de l'autre, un radical alkyle qui comporte 2 à 6 atomes de carbone ou un radical alkényle qui comporte 3 à 6 atomes de carbone ou CF₃.

8. Utilisation d'un ou de plusieurs composé(s) selon une ou plusieurs des revendications 1 à 7 dans des milieux cristallins liquides.

9. Milieu cristallin liquide comprenant au moins deux composés, **caractérisé en ce qu'**il comprend au moins un composé de la formule I : 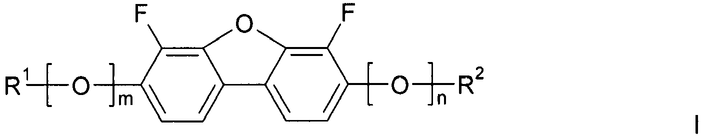 formule dans laquelle :
m et n sont chacun égaux à 1 ; et
R¹ et R² représentent, indépendamment l'un de l'autre, un radical alkyle qui comporte 1 à 15 atome(s) de C ou un radical alkényle ou alkynyle qui comporte 2 à 15 atomes de C, lesquels sont dans chaque cas en option monohalogénés ou polyhalogénés,
étant entendu qu'un mélange de la composition :
| Composé | % en poids |
|---|---|
| CY-3-O2 | 12,5 |
| CCY-3-O1 | 9 |
| CCY-3-O2 | 11 |
| CCY-4-O2 | 7 |
| CPY-3-O2 | 3 |
| CC-3-V | 31 |
| B-2O-O5 | 4 |
| PY-V2-O2 | 5,5 |
| CPY-V-O2 | 6 |
| CPY-V-O4 | 5 |
| CCY-V-O2 | 6 |
est exclus.

10. Milieu cristallin liquide selon la revendication 9, **caractérisé en ce que**, dans la formule I :
R¹ et R² représentent, indépendamment l'un de l'autre, un radical alkyle qui comporte 2 à 5 atomes de C.

11. Milieu cristallin liquide selon la revendication 9, **caractérisé en ce qu'**il comprend au moins un composé de la formule I qui est sélectionné parmi les composés des formules :

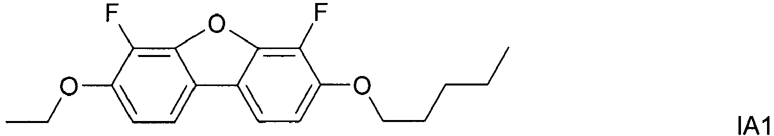

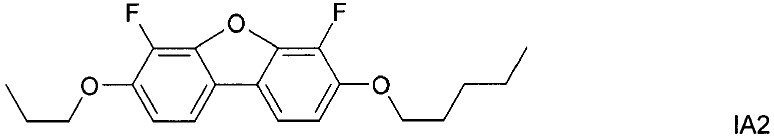

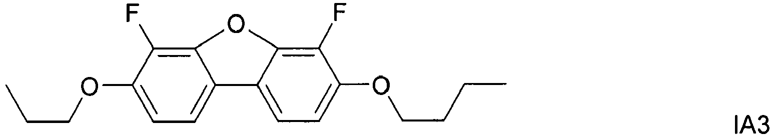

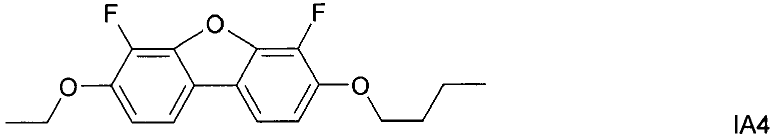

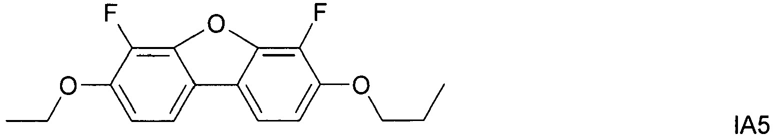

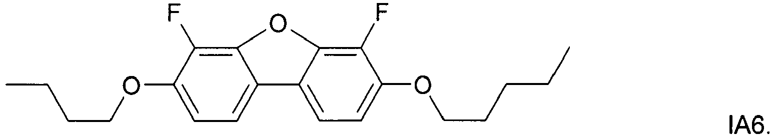

12. Elément d'affichage électro-optique contenant un milieu cristallin liquide selon la revendication 9.

13. Procédé pour la préparation de composés de la formule I selon une ou plusieurs des revendications 1 à 7, **caractérisé en ce qu'**il inclut une étape de procédé selon laquelle un composé de la formule (B) : 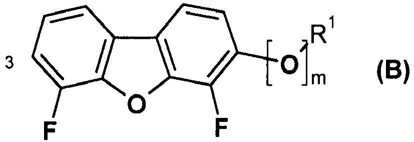 formule dans laquelle, de manière indépendante, m, R¹ sont définis comme selon la formule I selon une ou plusieurs des revendications 1 à 7,
est déprotoné à la position 3 au moyen d'un réactif de déprotonation et est converti selon un composé de la formule (C) : 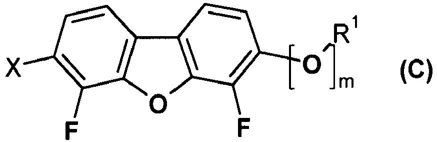 formule dans laquelle, de manière indépendante,
m, R¹ sont définis comme selon la formule I ;
X représente B(OR)₂, -C(OH)R ou OH ; et
R représente un radical alkyle qui comporte 1 à 14 atome(s) de C ;
et une ou plusieurs autre(s) étape(s) de procédé selon laquelle/lesquelles il est converti selon un composé de la formule I.
